# EUROPEAN PATENT APPLICATION

(11) **EP 1 772 825 A1**
(43) Date of publication of application: **11.04.2007**
(21) Application number: 05425699.5
(22) Date of filing: 06.10.2005
(51) Int. Cl.: G06T 7/00

(54) **Method for registering images of a sequence of images, particularly ultrasound diagnostic images**

(71) Applicant: Esaote S.p.A., 20100 Milano (IT)
(72) Inventor: Pedrizzetti, Gianni, 59100 Prato (IT); Tonti, Giovanni, 67039 (IT)
(74) Representative: Karaghiosoff, Giorgio Alessandro

(57) **Abstract**

Method for registering images of a sequence of images, particularly ultrasound diagnostic images and especially ultrasound diagnostic images of the heart. The said method comprises the steps of:
Providing at least a first and a second digital or digitalized image or set of cross-sectional images of the same object; Defining within one image a certain number of landmarks by selecting a certain number of pixels or voxels to be tracked; Tracking the position of each pixel or voxel selected from one to another image of said set of images by determining the relative displacements; Registering the set of images by applying the inverse displacement to the pixels or voxels between the images of said set of images.

Furthermore a region of interest, a so called ROI is defined by manually or automatically drawing a closed or open line on one image of at least two images, which line corresponds to the border of the said region of interest and one or more pixel or voxel which represents reference points to be tracked within the region of interest are selected; For each one of the said landmarks the displacement is estimated of the said landmark from one image to a following or previous image and calculating the amount of local displacement of the said landmarks from a first to a second image of the sequence of images and the morphological and motion evolution of the entire region of interest is reconstructed by means of the local displacements calculated for each landmark by applying the inverse evolution function of the morphological features and of the motion of the entire region of interest.

## Description

The invention relates to a method for registering images of a sequence of images, particularly ultrasound diagnostic images and especially ultrasound diagnostic images of the heart or another whole organ. The said method comprising the steps of:
a) Providing at least a first and a second digital or digitalized image or set of cross-sectional images of the same object, the said images being formed by a two or three dimensional array of pixels or voxels;
b) Defining within the first image or set of images a certain number of landmarks, so called features, by selecting a certain number of pixels or voxels which are set as landmarks or features and generating a list of said features to be tracked;
c) Tracking the position of each pixel or voxel selected as a feature from the first to the second image or set of images by determining the displacements from the first to the second image or set of images for each pixel or voxel selected as a feature;
d) Registering the first and the second image or set of images by applying the inverse displacement to the pixels or voxels of the second image or set of images.

Particularly relevant for obtaining good results is the step of selecting and tracking the landmarks recognized as features to be tracked.

The automatic tracking of objects is a fundamental topic in image analysis. In medical imaging the ability to automatically follow an organ would eventually facilitate the extraction of objective measurements and automate some diagnostic process.

Automatic tracking is useful for two principal aspects.

First, sometime it is required to extract an information from inside a region of a moving object. A driving example is the perfusion analysis by echography recording in presence of a contrast agent. Here the contrast uptake dynamics (wash-in and wash-out curves) reflects the microcirculation ability to perfuse the tissue and, as such, it is a fact intimately related with the organ function. Such a perfusion dynamics is measured in terms of the changes in the local brightness that must be evaluated on points that continuously belong to the same organ region, the same portion of tissue, even when the organ moves and presents a displacement from frame to frame. Other example are numerous like the Integrated BackScatter (IBS) level in cardiac tissue that is correlated with presence of collagen; in a different field, the change of temperature on top of moving clouds, in meteorological satellite imaging, is related to the evolutionary strength of stormy clouds. A tracking strategy is necessary to allow extraction of such quantities over the regions of object that does not occupy fixed points on the different images of a sequence. Such objects can move rigidly in the space, thus being traceable by following its center. Or the region of interest may be subjected to a more or less complex deformation that requires a correspondingly more or less complexity of the tracking method.

Secondly, the ability to track the movement of a region allows to evaluate information about its kinematic or dynamic properties, that may be related to relevant characteristics. Again a driving example is the movement of cardiac tissue: the trajectory of material points, as well as the relative movement of two or more material points, is a measure of the ability of the cardiac muscle to contract and relax and therefore a quantification of its function.

Landmark selection and tracking is known in combination with image registration processes. Considering a sequence of images which has been taken at different time instants then a certain number of landmarks are chosen in the first image. Each landmark corresponds to a point or a pixel or voxel in the first image.

These N points are defined by their position Xi with i=1...N. and X is a vector containing all the single scalar coordinate components. In two-dimensional images X is a pair of coordinates X=(x,y); typically x and y indicate row and column, or abscissa and ordinate.

Tracking consists in the following process:
Step II: Track the reference points.
   For each one of the reference point, the displacement of the point from one frame to a following or previous one is estimated, starting from the reference frame.

Herewith the term frame it is intended each image of the sequence, since an image sequence is like a cinematographic sequence.

For this purpose a small region about each of the points is automatically chosen, the extension of such region being defined as appropriate for the application, the expected entity of displacement, the quality of the images. The amount of local displacement between a pair of images is estimated by determination of the maximum likelihood between two such regions, in the two images, one region displaced of such amount relatively to the other region.

Several methods are available to define such optimal displacement. They have been used, in several different formulations, in many research fields. The so-called Particle Image Velocimetry (PIV) has been used in challenging conditions like those found in fluid turbulence. (Adrian RJ. Particle-image technique for experimental fluid mechanics. Ann. Rev. Fluid Mech. 1991;23:261-304) .

The general category known as Optical Flow, is commonly employed in advanced image analysis, (Singh A. Optic Flow Computation: A Unified Perspective. Piscataway, NJ: IEEE Comput. Soc. Press, 1992; Barron JL, Fleet DJ, Beauchemin S. Performance of optical flow techniques. International Journal of Computer Vision 1994;12:43-77) and are sometime referred as Speckle Tracking in echographic imaging.

From documents EP 1520517 and EP 1522875 a method is known for evaluating velocities in echographic imaging which provides also tracking steps for determining displacements and/or deformation of imaged objects or parts thereof during an image sequence, where the images are taken at different subsequent time instants. Velocity is determined in principle in dividing displacement by the time during which the displacement has occurred.

The displacement can be evaluated in order to be congruent with the evolutionary properties of the image sequence, if it is a periodic process, an average steady process, or a process with some known average properties.

The result of this step is the estimated displacement of each reference point ΔXi(t), with i=1...N, and where t indicates the time-frame and the displacement can be 'absolute' : with respect to one same frame for all frames, or 'relative' : with respect to frame that is distant a predefined time-delay (for example, often, the previous frame).

Relating to the method of estimating tissue velocity vectors and oriented strains from ultrasonic image data according to EP 152051, the method provides for the following steps:
Acquiring ultrasound image data from an object by transmitting ultrasound beams against the said object and receiving the corresponding reflected beams by the said object;
Determining the direction of motion and the velocity vector of the said reference points from the ultrasonic image data;
the ultrasonic image data consisting in a sequence of at least two image frames, said images being two dimensional or three dimensional data and particularly B-mode grey scale echographic images;
The velocity motion of each reference point between two successive B-mode image frames are determined by applying a so called particle image velocimetry technique abbreviated as PIV or another method in the class of Optical Flow methods;
Any component of the strain is then obtained from time integration of the strain-rate, that is evaluated from the gradient of velocity estimated form the velocity data in two or more points, or by measuring the deformation, typically shortening and lengthening, of a region of tissue between a pair or a group of tracked material points.

The method described in EP 1522875 also provides for tracking of points or landmarks in an image sequence.

According to EP 1522875, the tracking of position and velocity of objects' borders in two or three dimensional digital images, particularly in echographic images comprises the following steps:
Acquiring a sequence of at least two consecutive ultrasound image frames of a moving tissue or a moving object which ultrasound image frames are timely separated by a certain time interval;
Automatically or manually defining a certain number of reference points of a border of a moving tissue or object at least on a first image frame of the sequence of image frames acquired;
Automatically tracking the borders of the moving tissue or object in the at least one further following frame by determining the new position of the reference points of the border in at least one following image frame of the sequence of image frames by estimating the position of the said reference point in the said at least following image frames of the sequence of image frames one the basis of the ultrasound image data of the acquired sequence of image frames.

The sequence of image frames acquired is a sequence of consecutive B-mode, grey-scale ultrasound images. On a first frame a border line is drawn either manually or by means of an automatic border detection algorithm, the border being defined by a trace of pixels of the image frame coinciding with the said border line. The original trace of pixels coinciding with the manually or automatically drawn borderline is followed in time, i. e. in the at least ore following image frame, by searching the maximum likelihood of the trace of pixels in the following image frame with the trace of pixels in the first or timely previous image frame of the sequence of image frames by analyzing the image pixels in the neighborhood of the said trace of pixels.

According to a special embodiment of the technique disclosed in EP 1522875, the tracking of the border line is carried out by defining a certain number of reference points on the manually or automatically drawn border line on the first image frame and by using the method of transmural cuts. This transmural cuts are disclosed in greater detail in document PCT/IT02/00114 filed on 27.02.2002 and the transmural cuts consist in defining a line which crosses the borderline drawn and passing through one reference point. A physiologically appropriate direction can be chosen, which typically can be the orthogonal direction to the borderline at-the reference point.

The above disclosed procedure is a reduction of a two dimensional problem applied to a two dimensional image, such as a B-mode ultrasound image, to a one dimensional problem of an M-mode image. The tracking of the border line i.e. of the trace of pixels is carried out along the space- time image using across-correlation procedure of the pixel column in the space-time image corresponding to a first image frame with the pixel column in the space-time image corresponding to a successive image frame of the sequence of imago frames. This technique can be applied to any kind of image in which the geometry of the border line drawn does not require any kind of special reference points to be tracked a priori, such as for example closed borderlines of the cavity of a blood vessel in a cross-section image of the vessel.

When the object imaged has particular starting and ending points of a border which has a relevance as particular reference points in the motion executed by the border line of the object, for example in the case of the walls of the endoventricular cavity, then a preventive cycle can be carried out for optimally tracking the border line of the object along the sequence of image frames.

When the object has few very representative points the general topology of the border line of the object imaged can be represented by tracking the motion of these few representative points prior to carry out the tracking of at least one or some of the reference points lying on the manually or automatically drawn border-line in each frame of the sequence of image frames.

These representative points can be for example the starting and ending points of the border line when this is an open one.

The representative reference points of the border of an imaged object can be also suggested by the physiology when the imaged object is a particular tissue or organ, such as for example the left ventricle.

Thus prior to carry out the tracking of some or all of the reference points chosen on the manually or automatically drawn border-line of the imaged object along the sequence of image frames the tracking of this few representative reference points is carried out.

The tracking of this few representative reference points is carried out in a identical way as the one disclosed above for the other reference points on the border-line drawn manually or automatically on the first frame by using the method of transmural cuts for constructing space-time images of each of the few representative reference points and determining the displacement of these points in each of the frames of the sequence of image frames by means of cross-correlation between each of the pixel columns with the successive pixel column corresponding to the pixels along the transmural cut across the same representative point in the different image frames of the sequence of image frames. The direction of the transmural cuts can be chosen as the orthogonal direction.

After having determined the displacement of the few representative reference points on the border-line of the imaged object in some or in all of the frames of the sequence of image frames, the position and the displacement of the other reference points on the border-lines at each image frame of the sequence of image frames are obtained by rescaling the originally drawn border-line in the first image frame in such a way to obtain in each image frame corresponding to a successive instant a topologically equivalent border line geometry with respect to the original border line. Typically this results in a translation of all points along the original border line.

This preliminary rescaling allows to keep the representative reference points always in the proper position in all frames of the sequence of image frames by rearranging the other reference points so that the representative reference points maintains the same meaning relatively to the object in all frames of the sequence of image frames.

Tracking method according to EP 1 520 517 and 1 522 875 are nevertheless not designed for carrying out image registration. For this process the optical flow methods are used because the tracking according to EP 1 520 517 and 1 522 875 are considered as being too specific for the processes to which they are originally destined.

The present invention aims to provide for an alternative registration method which allows to take care also of the specific anatomical and morphological features of the imaged anatomical districts or of the imaged organ, thus avoiding that registration is carried out only on landmarks which are chosen in an abstract way without considering reality.

As a further object the invention allows also to provide at the same time for more additional information than the current registration methods.

The present invention achieves the above mentioned aims by providing a registration method comprising the steps of:
a) Providing at least a first and a second digital or digitalized image or a set of cross-sectional images of the same object, the said images being formed by a two or three dimensional array of pixels or voxels;
b) Defining within the first image or set of images a certain number of landmarks, so called features, by selecting a certain number of pixels or voxels which are set as landmarks or features and generating a list of said features to be tracked;
c) Tracking the position of each pixel or voxel selected as a feature from the first to the second image or set of images by determining the displacements from the first to the second image or set of images for each pixel or voxel selected as a feature;
d) Registering the first and the second image or set of images by applying the inverse displacement to the pixels or voxels of the second image or set of images.
   According to the present invention, tracking is carried out by means of the following steps:
e) defining a region of interest, a so called ROI, by manually or automatically drawing a closed or open line on the first image of the sequence of at least two images, which line corresponds to a trace of pixels or voxels of the said image and delimits the border of the said region of interest;
f) Defining one or more pixel or voxel which represents reference points within the region of interest defined by the said closed or open line and which reference points are the features or landmarks to be tracked;
g) for each one of the said features or landmarks estimating the displacement of the said feature or landmark from one image to a following or previous image starting form the first image by choosing a limited image region around the pixels or voxels defined as the landmarks or features and calculating the amount of local displacement of the said landmarks or features from the said first to said second image of the sequence of images by determining the maximum likelihood between the said limited regions in the first and in the second image;
h) reconstructing the morphological and motion evolution of the entire region of interest by means of the local displacements calculated for each landmark or feature.
i) applying the inverse evolution function of the morphological features and of the motion of the entire region of interest to the second or to a following image of the sequence of images.

Several methods are available to define the optimal displacement of the landmarks or features from one first image to a second or following image of the sequence. These methods have been used, in several different formulations, in many research fields. The so-called Particle Image Velocimetry (PIV) has been used in challenging conditions like those found in fluid turbulence. A detailed description is disclosed in Adrian RJ. Particle-image technique for experimental fluid mechanics. Ann. Rev. Fluid Mech. 1991;23:261-304.

The general category known as Optical Flow, is commonly employed in advanced image analysis, and is disclosed in Singh A. Optic Flow Computation: A Unified Perspective. Piscataway, NJ: IEEE Comput. Soc. Press, 1992 and Barron JL, Fleet DJ, Beauchemin S. Performance of optical flow techniques. International Journal of Computer Vision 1994;12:43-77.

Optical flow methods are sometime referred as Speckle Tracking in echographic imaging. A method for evaluating velocities in echographic imaging is described also in EP 1 520 517 or 1 522 875..

The displacement can be evaluated in order to be congruent with the evolutionary properties of the image sequence, if it is a periodic process, an average steady process, or a process with some known average properties.

The result of this step is the estimated displacement of each reference point ΔXi(t), with i=1...N, and where t indicates the time-frame and the displacement can be 'absolute' : with respect to one same frame for all frames, or 'relative': with respect to frame that is distant a predefined time-delay (for example, often, the previous frame).

According to a first improvement, the method of the present invention provides for differentiated determination of the so called rigid translation and rotation of the entire region of interest and of the deformations of the entire region of interest.

The evolution of the entire ROI (region of interest) defining the organ/object under analysis, that is not a point-wise element but has an extension, is made of a combination of several dynamical movements like translation, rotation, deformations. These can be reconstructed on the basis of the computed displacements of the reference points in a conceptually same manner.

The rigid translation, the ROI displacement along the coordinates, the vertical and horizontal displacements in two-dimensional images, is evaluated by the displacements of the reference points by extracting their statistical most significant measure; for example the mean value or a weighted average, or the mean of the non extreme values, or the median, or another appropriate measure that may depend on the specific application.

The rotation about a given point can also be evaluated from the statistical significant measure of the rotation of each reference point i.e. of each landmark or feature. The deformation, as well as any other evolutionary characterization, can be equally extracted by evaluation of the corresponding relative displacements of the reference points.

In general the information about the landmarks or features motion must be sufficient to evaluate the location of the region of interest (ROI) from one image to any other in a way that the region of interest (ROI), that moves, rotates, and deforms, can be identified on any image or frame of the sequence.

An improvement of the method according to the present invention consist in the fact that the line or the lines defining the border of the region of interest to be tracked is a line coinciding with an edge or a border of the image of an anatomical object or of an organ.

The pixels or voxels defined as reference points which has to be considered as landmarks or features to be tracked by a first reference image to a second or a following one of the sequence of images or frames are at least partly or entirely (all) chosen as lying on the said line defining the border of the image of the anatomical object or of an organ.

In this case the border line is tracked and allows to determine rigid motion as well as deformation by means of the method disclosed in EP 1 522 875 where the landmarks or features defined on the border line are tracked by means of the so called transmural cut technique. A short disclosure of this technique being given in the introduction of the present description.

Transmural cuts consist in lines which are generally straight, but which can also be curved ones and which are oriented transversally to the border line defining the region of interest, passing through the pixel or reference point on the said border line defined as a landmark or feature to be tracked.

The orientation of the said transverse lines are chosen according to an expected motion of the feature or landmark the said line describing an estimated path.

This operation is made for each image frame of the sequence of image frames and for each reference point chosen as a feature or landmark to be tracked.

The pixels taken along each transmural line in each of the image frames of the sequence of image frames are placed in columns, each column corresponding to one image frame of the sequence of images. In this way the evolution along a transmural cut, can be represented for all instants at once in a two-dimensional space time representation.

The above disclosed procedure is a reduction of a two dimensional problem applied to a two dimensional image such as a B-mode ultrasound image to a one dimensional problem as a M-mode image.

The tracking of the border. i.e. of the trace of pixels is carried out along the space-time image using a cross-correlation procedure or another maximum likelihood estimate of the pixel column in the space-time image corresponding to a first image frame with the pixel column in the space-time image corresponding to a successive image frame of the sequence of image frames.

This technique can be applied to any kind of images in which the geometry of the border line drawn does not require any kind of special reference points to be tracked a priori such as pro example closed border lines as the border line of the cavity of a blood vessel in a cross-section image of the vessel.

When the object imaged has particular starting and ending points of a border which has a relevance as particular reference points in the motion executed by the border-line of the object, for example in the case of the walls of the endoventricular cavity, then a preventive cycle must be carried out for optimally tracking the border-line of the object along the sequence of image frames.

According to a further improvement of the above mentioned method when the object has few very representative points the general topology of the border line of the object imaged can be represented by tracking the motion of these few representative points prior to carry out the tracking of at least one or some of the reference points lying on the manually or automatically drawn border-line in each frame of the sequence of image frames.

These representative points can be for example the starting and ending points of the border line when this is an open one.

The representative reference points of the border of an imaged object can be also suggested by the physiology when the imaged object is a particular tissue or organ, such as for example the left ventricle.

Thus prior to carry out the tracking of some or all of the reference points chosen on the manually or automatically drawn border-line of the imaged object along the sequence of image frames the tracking of this few representative reference points is carried out. The tracking of this few representative reference points is carried out in a identical way as the one disclosed above for the other reference points on the border-line drawn manually or automatically on the first frame by using the method of transmural cuts for constructing space-time images of each of the few representative reference points and determining the displacement of these points in each of the frames of the sequence of image frames by means of cross-correlation between each of the pixel columns with the successive pixel column corresponding to the pixels along the transmural cut across the same representative point in the different image frames of the sequence of image frames.

The direction of the transmural cuts can be chosen as the orthogonal direction

According to a further feature after having determined the displacement of the few representative reference points on the border-line of the imaged object in some or in all of the frames of the sequence of image frames, the position and the displacement of the other reference points on the border-lines at each image frame of the sequence of image frames are obtained by rescaling the originally drawn border-line in the first image frame in such a way to obtain in each image frame corresponding to a successive instant a topologically equivalent border line geometry with respect to the original border line. Typically this results in a translation of all points along the original border line.

This preliminary rescaling allows to keep the representative reference points always in the proper position in all frames of the sequence of image frames by rearranging the other reference points so that the representative reference points maintains the same meaning relatively to the object in all frames of the sequence of image frames.

Thus in a preferred embodiment the complete method according to the invention comprises the following steps:
a) Acquiring a sequence of at least two consecutive ultrasound image frames of a moving tissue or a moving object which ultrasound image frames are timely separated by a certain time interval;
b) Tracing a border line over one single first frame either manually or with the help of an automatic border drawing algorithm;
c) Tracking the position displacements of one or more eventually present representative reference points over the entire sequence of consecutive image frames;
d) Rescaling the border line drawn on the first image frame at least for some or for each of the following image frames of the sequence of image frames according to the corresponding position tracked of the representative reference points;
e) Defining a certain number of further reference points distributed along the border line on the first image frame and falling on the said border line;
f) Tracking the position of each point independently from the others along the sequence of image frames;
g) Tracking of the position of the representative reference points and of the other reference points being carried out by
h) for each point independently and in each of the image frames of the sequence of image frames defining a transmural cut line consisting in a line which crosses the border line drawn and passing through the said reference point;
i) the pixels taken along each transmural cut line in each of the image frames of the sequence of image frames are placed in columns, each column corresponding to one frame of the sequence of images for representing the evolution along a transmural cut line, for all instants at once in a two-dimensional space time representation;
j) the tracking of the border. i.e. of the trace of pixels along each transmural cut line is carried out along the space-time image using a cross-correlation procedure or another maximum likelihood estimate of the pixel column in the space-time image corresponding to a first image frame with the pixel column in the space-time image corresponding to a successive image frame of the sequence of image frames.

According to an improvement in both cases disclosed above when images are poor with a low signal-to-noise ratio the space-time representation along the transmural cuts can be built using a line for the transmural cut with a thickness larger than that of a single pixel and by extracting the average value across such a thickness.

The above mentioned method can be further developed for carrying out a surface border tracking three dimensional imaging.

The method according to the said development comprises the following steps:
l) Acquiring a sequence of three-dimensional ultrasound imaging data sets, each three-dimensional data set being acquired with a predetermined time interval from the previous one;
m) Defining at least a principal section plane of each three dimensional data set along one chosen direction for obtaining a sequence of two dimensional image frames along the said section plane;
n) Drawing a border line of the object imaged either manually or automatically on the first two dimensional image frame of the sequence of two dimensional image frames taken along the said section plane;
o) Carrying out the tracking steps c) to j) previously disclosed for the two dimensional sequence of image frames;
p) For each three dimensional data set of the sequence of three dimensional datasets defining a preestablished number of further secondary section planes crossing the at least one, preferably all the principal section planes, the said secondary section planes being spaced apart one from the other along a predetermined direction and dividing the object represented by each three dimensional data set of the sequence of three-dimensional data sets in slices.
q) For each secondary section plane in the sequence of three dimensional data sets constructing the corresponding sequence of image frames relative to the said secondary section plane;
r) For each sequence of two dimensional image frames determining a guess border line in one single frame, by letting the border line passing across the intersection points with the principal section planes;
s) Tracking the said guess border line by detecting a new border by applying the method steps according to c) to j) or e) to j) by substituting the time coordinate in the said disclosed steps with the spatial coordinate along the said guess border line by
t) defining a certain number of transmural cuts on the single image of the sequence of images along the guess border line;
u) identifying the pixels along the said transmural cuts and placing the pixels along each transmural cuts side by side for constructing a two dimensional image where the horizontal axis indicates the spatial coordinate along the guess border line;
v) carrying out the cross correlation between each of the consecutive pixel columns in the said two dimensional image and thus tracking the border line in one frame for each of the sequences of two dimensional image frames corresponding to each of the secondary section planes.

According to a further improvement one or more further principal section planes can be defined along each of which further section planes the methods steps 1) to o) are carried out.

In a preferred embodiment two orthogonal principal section planes are chosen for carrying out the above mentioned method steps, the crossing line of the two principal section planes defining a preferred direction of the said planes.

The said direction can be chosen as suggested by the topological or functional feature of the object imaged.

Considering ultrasound images of a biological tissue or of an organ such as for example the heart the said direction can be suggested by physiological reasons. For example this physiologically relevant direction can be chosen as the cut across a central vertical plane such as the ventricle axis.

In order to better define the share of secondary section planes cutting the principal section planes, the method according to the present invention comprises the steps of defining bounds or limits for a distance range within which the share of the said secondary section planes is defined.

Preferably a topological or physiological relevant direction is chosen, particularly the same direction defined for determining the principal section planes, along which direction bounds are determined for the ends of a distance range within which the share of secondary section planes at least transversal, particularly perpendicular to the said relevant direction is determined.

Still according to a further embodiment, when determining the first guess border line, this one is determined as a physiologically relevant line passing through the reliable points.

According to the present border tracking method the correct border is determined along a sequence of two dimensional or three dimensional ultrasound image data and the correct border for each image frame can be displayed overlaid on the displayed image frame as an highlighted line characterized by a color which is different from the grey-scale B-mode image displayed.

By means of the above tracking method of the border of two dimensional images or three dimensional images of an object it is also possible to determine the border line velocity and thus the border velocity of the object imaged.

Once that the displacement of the border line is known along the sequence of two dimensional or three dimensional ultrasound image data sets and the time interval between each two consecutive sets of image data are known the component(s) of velocity in the direction of the transmural cut(s) can be estimated for each reference point by means of a simple calculation.

The complete velocity vector is determined by evaluation of the other component(s) of velocity, the total number of components being two for two-dimensional imaging and being three for three-dimensional imaging.

Each missing component of velocity can be evaluated once again with the method of transmural cuts.
aa) For each reference point, on each image frame of the sequence of image frames, a transmural cut consisting in a line which crosses the tracked point and directed along the direction where the additional component of velocity must be evaluated, typically orthogonal to the direction previous employed for tracking the border.
bb) the pixels taken along each transmural cut line in each of the image frames of the sequence of image frames are placed in columns for all instants at once in a two-dimensional space time representation;
cc) the evaluation of the velocity component along the chosen direction is carried out along the space-time image using a cross-correlation procedure of the pixel column in the space-time image. The velocity being given by the ratio of the column-wise displacement of the correlation maximum and the time interval between the corresponding frames.

The method is identical of that employed for tracking the border with the difference that only the frame-by-frame displacement is required and the eventual time integration of said displacement to get the motion of the border is ignored.

A different evaluation of the velocity vector can be obtained by applying two dimensional-correlation techniques or a specific optical flow technique particularly developed for ultrasound image data of moving objects.

The said velocity estimation method can be carried out in combination with the above disclosed method for tracking the border of the imaged moving object.

The said method is an adaptation of known method so called OPTICAL FLOW methods, like a known method so called PIV method used in fluid dynamics.

Thus in displaying the B-mode images the border tracked can be drawn as a line as disclosed above and the velocity vectors of the border taken at certain number of points of the said border line are displayed as arrows having a different color as the border line and the direction of the velocity vector and a length corresponding to the modulus of the velocity vector in the image plane of the two dimensional image displayed.

By means of the method according to the present invention a different approach for tracking borders of a moving object in two and three dimensional ultrasound imaging is provided where the borders are not "detected", rather they are "tracked", i.e. followed in time, starting from one reliable existing instantaneous trace that is commonly -but not necessarily- manually drawn by the experienced operator over one single frame. Using this approach all the ambiguousness that are present in a pure detection approach are cleared, the original trace is followed in time by searching the maximum likelihood over its neighbourhood in the following frames. The tracking technique for each single point is approached using a method based on transmural cuts that is similar to that introduced in the document PCT/IT02/00114 filed on 27.02.2002. Afterward the velocity on the tracked borders are estimated on the basis of the same maximum likelihood between two consecutive frames.

The automatic tracking method disclosed here allows the tracking of a border on a sequence of two-dimensional or three-dimensional images, and the evaluation of the velocity vector field on such borders. In principle, the border could be tracked on the basis of the velocity vector only, however a tracking procedure is a result of the summation (time integration) of the estimated velocities and is prone to an error growth in presence of small incorrect estimates. This approach reduces the two- or three-dimensional tracking to a combination of one-dimensional tracking problems along the single topological relevant direction (typically the orthogonal to the border), that can be much better controlled and made accurate. On the opposite, the accurate tracking result is employed to improve the estimates of the velocity vector.

The result of this procedure is the automatic definition of the borders displacement and velocity over all frames of a sequence of images, starting from the border traced on a single image.

Eventually, the found borders information will be used to evaluated some geometric properties, like volume, area, or sizes, of the organ. The border kinematics (tracking + velocity) allows to estimate global quantities (like volumes, lengths) as well as local phenomena (like rotations, strain) in a unique approach.

According to the present invention the found borders information, i.e. displacement and velocity vectors will be used to generate inverse displacement and velocity vectors in order to transform back either rigid displacement of the region of interest, i.e. the organ or the like and also deformations of the region of interest occurred during the global acquisition time of the sequence of images. This step is useful for comparing the different images of the sequence of images relating to changes in brightness of the pixels or voxels representing the imaged region of interest and particularly in the so called perfusion measurements, where the wash in and wash out of a contrast agent in the relevant region of interest has to be measured.

Perfusion measurements consist normally in acquiring at different time instants an image of the same object. A first image, so called reference image is taken when a specified amount of contrast agent, like no amount or maximum amount, is present in the imaged object. The following images of the sequence are taken after contrast agent is present in the imaged object. Since contrast agent presence influences the brightness of the image pixels a measure of the velocity of perfusion of the said contrast agent can be achieved by comparing the brightness, for example the means brightness of the region of interest in the different image frames of the sequence of images.

This comparison is carried out by comparing each of the images of the sequence taken at different time instants with the reference image taken when a certain amount of contrast agent is present in the region of interest.

The particular tracking method used in the present invention allows also to determine further information. Since for each landmark or feature to be tracked the displacement in time is determined it is possible to calculate and draw on any image frame of the sequence of images the path along which the said landmarks or feature has moved during the acquisition time of the sequence of images.

This information can be useful for evaluating the different time behaviours of local limited regions of the complete region of interest and thus of the anatomical part or of the organ represented by the said region of interest. So for example, considering heart motion it is possible to visualize the path of motion of the single points of the ventricular wall coinciding with the landmarks or features that has been tracked according to the above disclosed method.

### BRIEF DESCRIPTION OF THE DRAWINGS

Further improvements of the present invention are subject matter of the dependent claims.

The features of the invention and the deriving advantages will disclosed with greater detail in the following description of some examples and by means of the accompanying drawings in which:
Figure 1 illustrates an echographic image of the left ventricle, in long axis view (from the apex to the mitral plane), extracted from an echocardiographic recording (58 frames, 2 cycles) with an endocardial border (white) and the instant border velocity (dark white arrows).
Figure 2 illustrates an echographic image of the left ventricle, in short axis view (transversal section), extracted from an echocardiographic recording (49 frames, 2 cycles) with a "closed" endocardial border (white) and the instant border velocity (dark white arrows).
Figure 3 illustrates an echographic image of the left ventricle, in long axis view. The traced endocardial border implicitly defines the starting and final points and the mitral plane. The position of the transmural cuts, passing from the edge points and normal to the transmitral plane, are indicated.
Figure 4 illustrates a space-time representation, where space is along a transmural cut, of the echographic images sequence. The transmural cut is taken as for the starting point in figure 3. The time evolution of the starting point, tracked automatically, is reported.
Figure 5 illustrates an echographic image of the left ventricle, in long axis view, during diastole (left) and systole (right). The original endocardial border just rescaled on the based of the mitral plane displacement is shown. The instantaneous transmural cuts on each of the originally points are also drawn.
Figure 6 illustrates a schematic view of an example of cutting a three-dimensional dataset with two orthogonal planes, having one common direction, to get two two-dimensional images
Figure 7 illustrates a schematic view of an example of cutting a three-dimensional dataset with transversal planes that are orthogonal to the principal planes like those employed in figure 6.
Figure 8 illustrates slices on a three-dimensional echographic dataset of the left heart. The left ventricle border is known on the vertical image. The border at the intersection with the horizontal slices represent the starting points for tracking the border on the horizontal images.
Figure 9 illustrates an horizontal slice on a three-dimensional echographic dataset of the left heart. The starting points and a circular first guess border are indicated.
Figure 10. is an echographic image of a kidney during contrast agent infusion. The kidney undergoes to small movements due to heartbeat, respiration, and handheld transducer
Figure 11 is an echographic image of a kidney during contrast agent infusion. The dashed curve defines a region of interest that contains the organ; the crosses defines reference points used to track the region
Figure 12 shows the echographic image of a kidney at two instant during contrast agent infusion. In the left image the continuous line defines the region of interest. In the right image the kidney has moved and the previous ROI, dashed, moves to the new position, continuous curve, by horizontal and vertical displacements
Figure 13 illustrates the perfusion curve extracted from a large region of the myocardium, interventricular septum, as evaluated from echographic recording during contrast agent infusion and the curve which is the representation of perfusion in terms of a parametric curve of the type y=A*exp(-t/T) identified by the two parameters A and T
Figure 14 illustrate the parametric representation of perfusion, as evaluated from echographic recording during contrast agent infusion of a kidney (right image), and of a myocardium (left). The kidney presents a rigid displacement due to heartbeat and breathing, the myocardium is subjected to significant deformation during systole-diastole cycle. The parametric images of good quality have been possible after motion compensation.
Figure 15 illustrate the echographic image of the left ventricle at one instant. The white curves show the trajectory of 12 material points that at this frame are instantaneously located under the red circle.

### DETAILLED DESCRIPTION OF THE DRAWINGS AND OF THE METHOD

### Two-dimensional imaging

The method steps according to the present invention are firstly described with reference to a two dimensional case. A sequence of two dimensional B-mode image frames is acquired. The frames are acquired at predetermined time interval one form the other.

Consider a generic sequence of two-dimensional (2D) images comprising a certain number of two dimensional images each one taken at different following time instants; mathematically, this is a three-dimensional (3D) information that is 2D in space and 1D in time.

Consider that the images contain one organ/object or part of it, that changes its position and shape in time, of which organ we want to trace the border kinematics at all instants.

The method according to the present invention comprises a first step which consist in defining a region of interest (ROI) by tracing the border of the region of interest over one single frame by defining a border line.

A border is traced, manually or by another manual or automatic procedure, over one arbitrary frame ,preferably the first image of the sequence of images. Such border is then defined as a sequence of N points, defined by their coordinate pairs (xi,yi) with i=1...N in the two dimensional image plane.

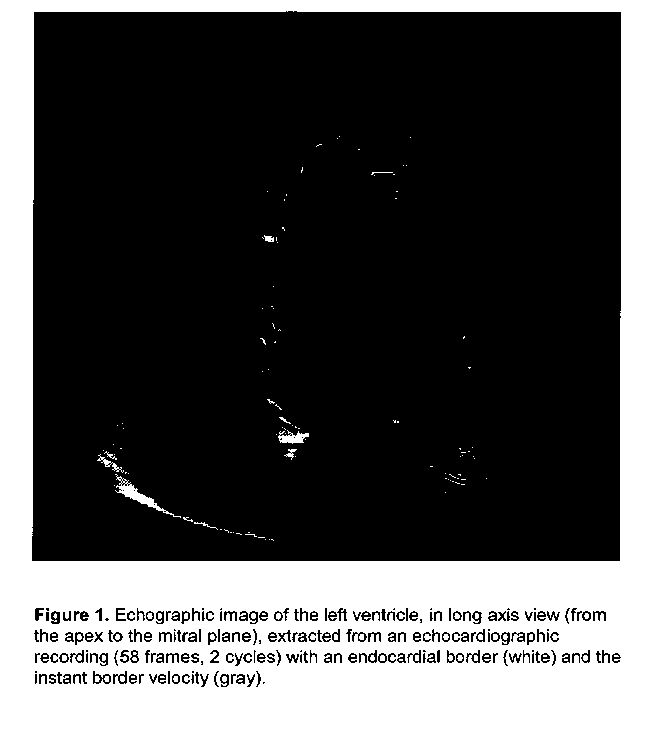

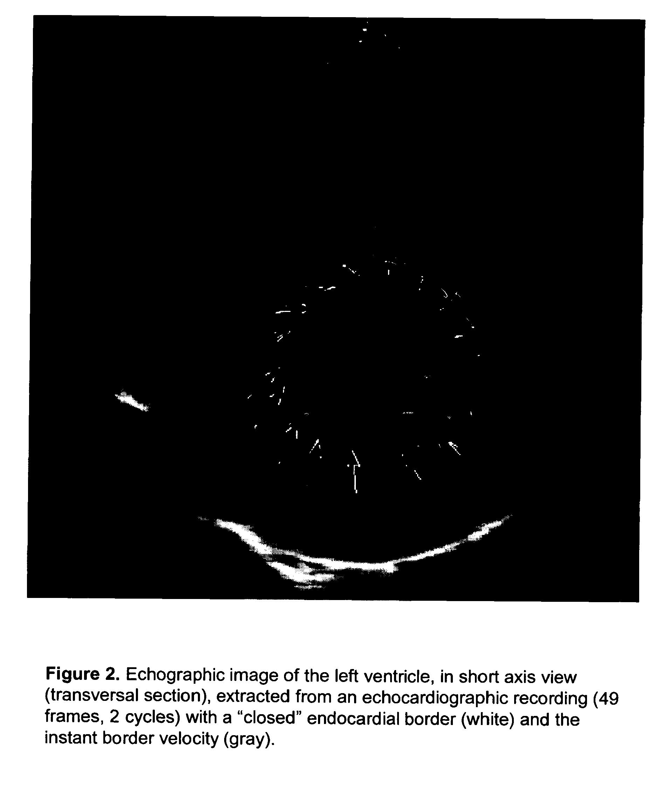

The result of this step is illustrated in figure 1 and 2. Figure 1 illustrates an image of the left ventricle where the endocardial border points are traced from one side of the mitral annulus to the other side of the same mitral annulus. Figure 2 illustrates an example in which the border is a closed one where the Nth point connects to the first one.

For the purposes of tracking the movements and the deformations of the organs imaged in the examples of figure 1 and 2,only reference points are chosen coinciding with the borderline, which reference points becomes landmarks or features to he tracked in the following images of the sequence of images. As it will become apparent in the following description the reference points care also be chosen inside the region delimited by the drawn borderline i.e. inside the ROI.

Referring now to a particular case like the one illustrated in figure 1, where the border has a staring and an ending point, the method according to the invention provides for a second step of tracking the most representative reference point of the border line drawn in the first image frame.

According to this step, the general topology of the object border is reproduced on all the images by tracking the motion of a few representative points. These are commonly the starting and final points of the border line when this is an open one. In specific cases additional reference points can be added to improve the first evaluation of the region about which the border must be tracked.

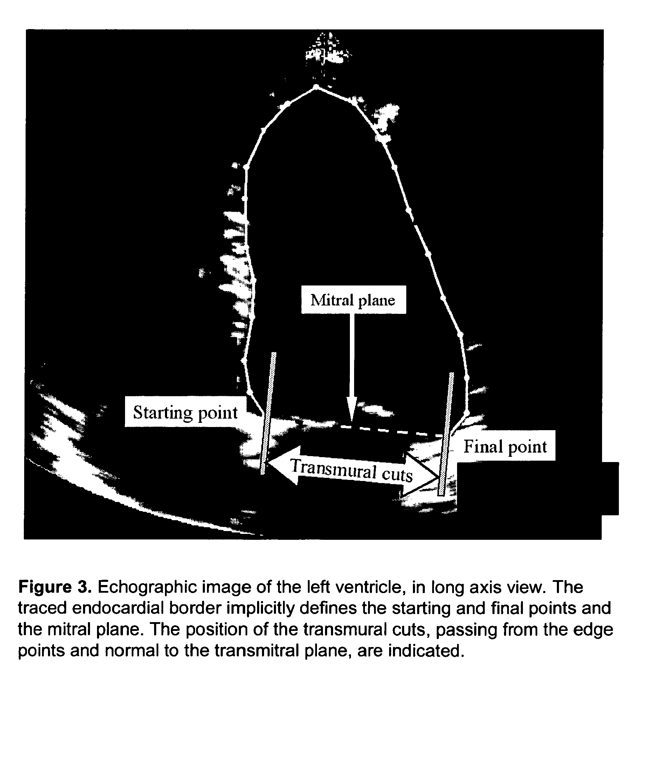

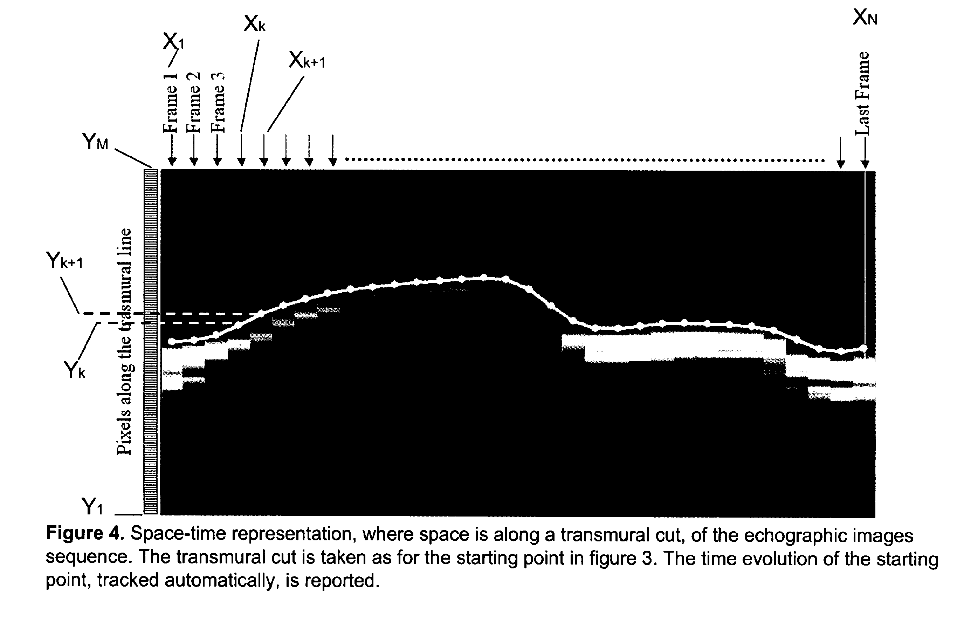

The displacement of the representative reference points, namely the chosen landmarks or features, along one or more specific directions from one image frame to a following image frame is evaluated by tracking. Figure 3 shows the reference points for a left ventricle (in long axis view) that are the starting and final points of the originally traced border. In this case, the physiology suggests to track the motion of these points in the direction instantaneously orthogonal to the mitral plane (that is defined by these points).

The tracking along a specified direction is performed by using the method of transmural cuts as follow. A line crossing the wall, passing through the reference point, and directed along the physiologically appropriate direction is drawn; in the case shown in figure 3 the appropriate direction is orthogonal to the mitral plane. In general two orthogonal direction can be employed. The pixels taken along the chosen direction line(s) are placed in columns, each column corresponding to one frame of the sequence of images. In this way the evolution along a line can be represented for all instants at once in a two dimensional space-time representation (sometime referred as M-mode representation) where one axis is the distance along the line and the other axis is the time. An example of such a representation is shown in figure 4.

In the case of poor images with a low signal to noise ratio the space time representation can be built using a line for the transmural cut with a thickness larger than that of a single pixel and extracting the average value across such a thickness.

The border tracking is then performed along the space-time image.

### Tracking along the 2D space-time image

The tracking procedure according to the present example is a procedure for following a border line along one direction in a two-dimensional image like that in figure 4 starting from a known position at one instant.

Let us call x the horizontal direction and y the vertical direction, and indicate with xi , i=1...N, where N is the number of columns in the image. The tracking is given by determination of a discrete sequence of real numbers yi=y(xi), starting from a known point yk corresponding to the columns xk.

This is a one dimensional tracking problem that can be solved with several possible standard methods. One method is reported here for giving completeness to the whole invention that can, however, employ also different techniques for this specific task when suggested by the specific imaging employed.

The displacement from the known point yk to the point yk+1 can be estimated by evaluating the cross-correlation between the entire column at xk with the entire column at xk+1. The cross-correlation function will present a maximum, the position of the maximum gives the value of the vertical displacement required to maximize the similarity between the two columns, therefore yk+1 is estimated by adding such a displacement to yk. This procedure is repeated between all pairs of nearby columns and the result is an estimate of the entire border yi , i=1...N.

In this procedure it is convenient to employ windowing techniques that avoid side effects given by the two ends of the finite size columns. When applicable, it is also convenient to make use of the periodicity of the signal along x in order to perform the method in Fourier space.

The first estimate is improved by applying the same procedure recursively on increasingly reduced spatial width about the previously found border.

This first estimate yi can be further improved. To this aim a subset of the image is extracted by taking a few points above and below the first estimate yi, and a new image whose center corresponds to the sequence yi is generated. A snake procedure like the one described in Blake A., Yuille A. Active Vision MIT press, 1992., is employed to follow, in the new image, the image brightness level that passes through the fixed point yk. As a result the estimation of yi, i=1...N is refined.

As it will become clear in the following description the tracking technique is a unique procedure that is common to different steps of the method according to the present invention.

As applied to the above mentioned step of the method according to the present invention, the result of this preliminary tracking procedure is the position and displacement, at all instants, of the most representative reference points along the predefined direction, or the vector combination when two directions are employed.

After this, all the other points of the original border are rescaled at each instant in order to get, at each instant a topologically equivalent border geometry. Typically, like in the example of figure 2, all the points are translated along the original curve.

This preliminary rescaling procedure permits to keep the reference points always at the proper position in all the frames, and to rearrange the other points so that the reference maintains the same meaning in all the frames.

The present step of tracking the most representative reference points such as the starting and ending point of a border line can be avoided when the specific geometry does not require or have any representative reference point to be tracked a priori. One example where this step can be avoided is given by the closed geometry in figure 2.

After having carried out the tracking of the most representative reference points if these points are present or in place of the said tracking step the method according to the invention provides for a further step consisting in the tracking of all the other reference point on the border line drawn manually or automatically in the first step on a first two dimensional image frame of the sequence of image frames.

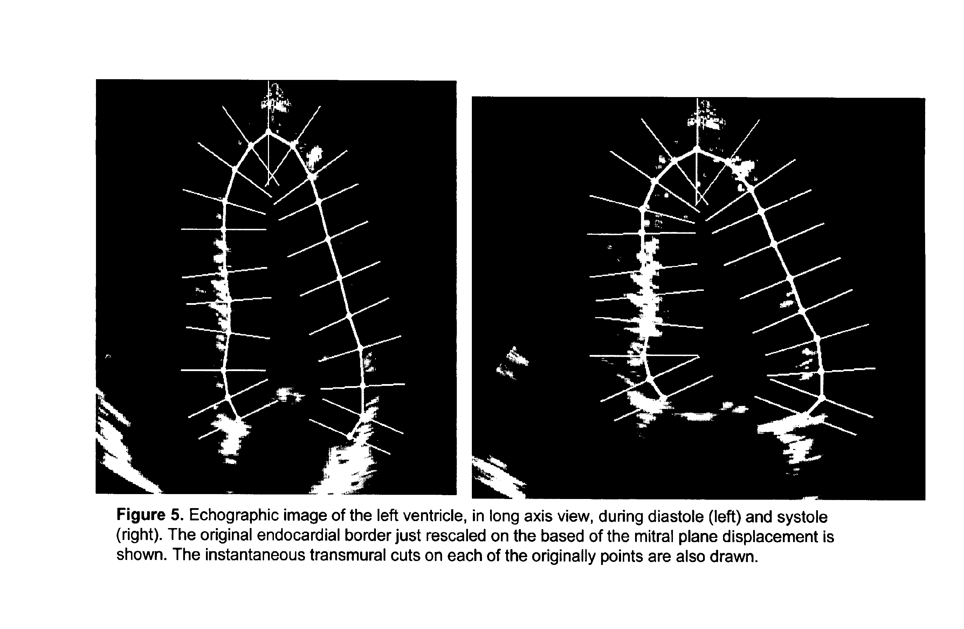

For each point, independently, the tracking along a specified direction is performed by using the method of transmural cuts as follow. A line crossing the wall, passing through the point, and directed along the physiologically appropriate direction is drawn, this operation is made for each instant/frame of the sequence of image frames because the points are not fixed in time but they have been previously rescaled at each instant accordingly with the instantaneous displacement of the reference points. In most cases, like in the case shown in figure 5, the appropriate direction is taken at each instant as orthogonal to the rescaled border. The pixels taken along each transmural line are placed in columns, each column corresponding to one frame of the sequence of images. In this way the evolution along a transmural cut, that is not fixed in all frames time but is slightly modified accordingly to the rescaling, can be represented for all instants at once in a two-dimensional space time representation analogous to that shown in figure 4.

In the case of poor images with a low signal-to-noise ratio the space time representation can be built using a line for the transmural cut with a thickness larger than that of a single pixel and extracting the average value across such a thickness.

The border tracking is then performed along the space-time image using the same technique employed in the step of tracking the representative reference points and disclosed above in a detailed manner.

The result of this step is the position, at all instants, of all the points along the predefined direction, or the vector combination when two directions are employed. At this stage all the original points have been tracked in time, each one independently, and we have a new border tracked over all frames.

It can be useful, especially in poor quality images, to improve the estimate by including a spatial coherence in the tracked border. This can be done by verifying the likelihood of the tracking between neighboring points and correcting the eventual discrepancies with appropriate filters or validation methods.

As an additional procedure the method according to the present invention can be provided in combination with a procedure for determining the instant border line velocity vector for each one of the reference points defined on the border line as tracked on each two dimensional frame.

For each point, independently, the velocity vector can be known when two direction (three for three-dimensional imagin) are employed for displacing it. When a single direction is employed, the complete velocity vector can be evaluated by selecting additional direction for the transmural cuts on the already displaced point and evaluating the velocity along the additional direction.

In the case of poor images with a low signal-to-noise ratio the space time representation can be built using a line for the transmural cut with a thickness larger than that of a single pixel and extracting the average value across such a thickness.

Alternatively, the complete velocity vector can be evaluated by a two-dimensional correlation technique or a specific optical flow technique adapted to the particular case of ultrasound imaging B-mode data. The two-dimensional result can then be improved by imposing its accordance with the previous estimate obtained for one component from the transmural cut approach. Results of the entire procedure are shown, for one frame, in figure 1 and 2.

### Three-dimensional imaging

The same steps described for the analysis of two-dimensional imaging can be employed for the border tracking in three-dimensional imaging. Such an extension is straightforward by using the previous steps in an appropriate combination, and substituting, in one case, the time direction with one spatial direction. Eventually no additional manual intervention is necessary with respect to what is done in two-dimensions, i.e. the indication of the border in one 2D frame.

A sequence of three-dimensional (3D) datasets is mathematically a four-dimensional (4D) information that is 3D in space and 1D in time. Consider that the images contain one organ/object or part of it, that changes its position and shape in time, of which organ it is desired to trace the border at all instants, the border now being a sequence of two-dimensional surfaces.

As a first step the method according to the present invention provides to choose one principal section plane which cuts to the three-dimensional dataset, and to apply the entire two-dimensional technique disclosed above on such plane.

The principal section plane of the 3D dataset is one plane, preferably along a physiologically relevant direction. Cutting the 3D datasets of the sequence of 3D datasets with this plane furnishes one sequence of 2D images.

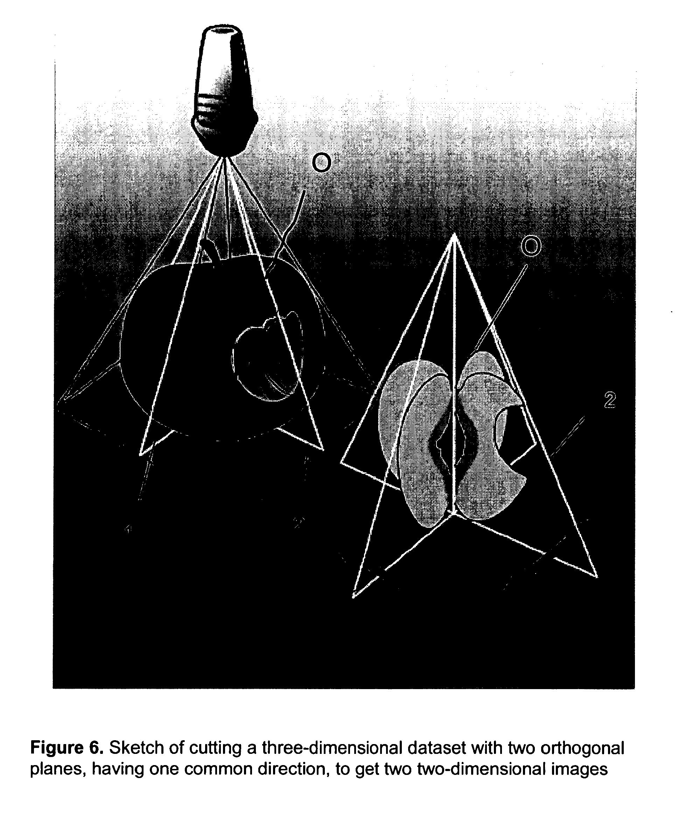

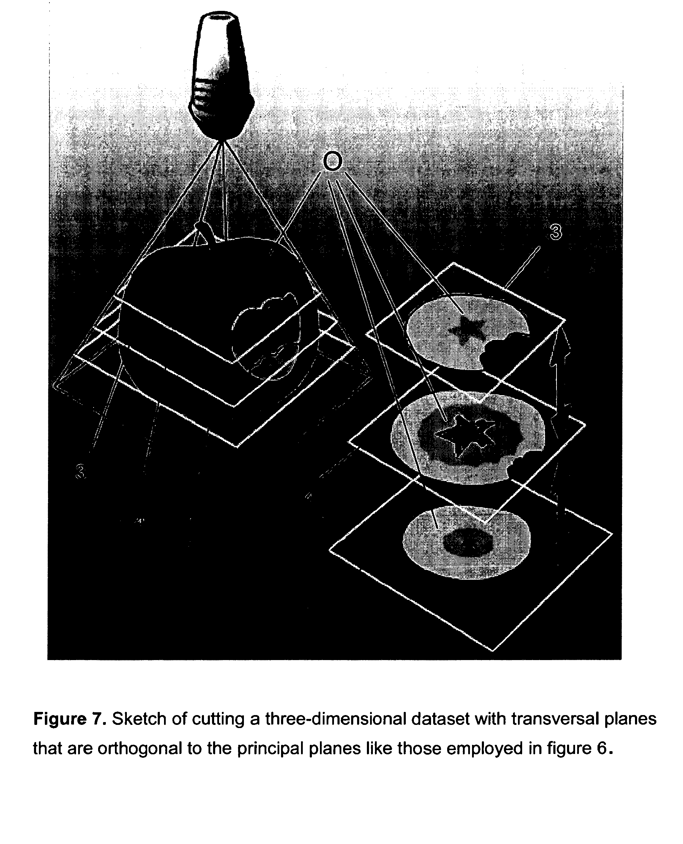

Figure 6 illustrates the cutting of a three-dimensional data set of ultrasound image data of the object 0 with two orthogonal principal section planes 1 and 2 oriented in the vertical direction.

For each principal section plane the entire tracking procedure as disclosed above for the sequence of two dimensional images is applied to this two-dimensional sequence of images taken on the principal section planes in order to track the border and evaluate the velocity on such principal section plane. This border is the signature of the sought border kinematics on the plane.

The above steps can be repeated with more than one or two principal section planes to improve the reliability of following steps in poor quality images.

After having carried out the above mentioned step a further step is carried out consisting in defining secondary section planes to the three-dimensional dataset, and applying the two-dimensional technique on single frames substituting the time direction with one spatial direction.

The previous step allows to define the bounds of the surface border. For this, one direction is chosen over the plane cut used in the previous step, preferably a physiologically relevant one (like the ventricle axis), and, for each instant, evaluate the upper and lower bounds along such direction of the border found in the previous step.

The range between these limits, at each instant, is further divided in M internal points, and the 3D dataset is cut in correspondence of such M points, with M secondary section planes that are orthogonal to the chosen direction as indicated with 3, 4, 5 in figure 7. By means of the said secondary section planes the corresponding M sequences of 2D images are constructed.

Successively, for each sequence, a reliable border in one single frame is defined, commonly the same frame used when the borders are drawn manually during the previous step relative to the principal section planes.

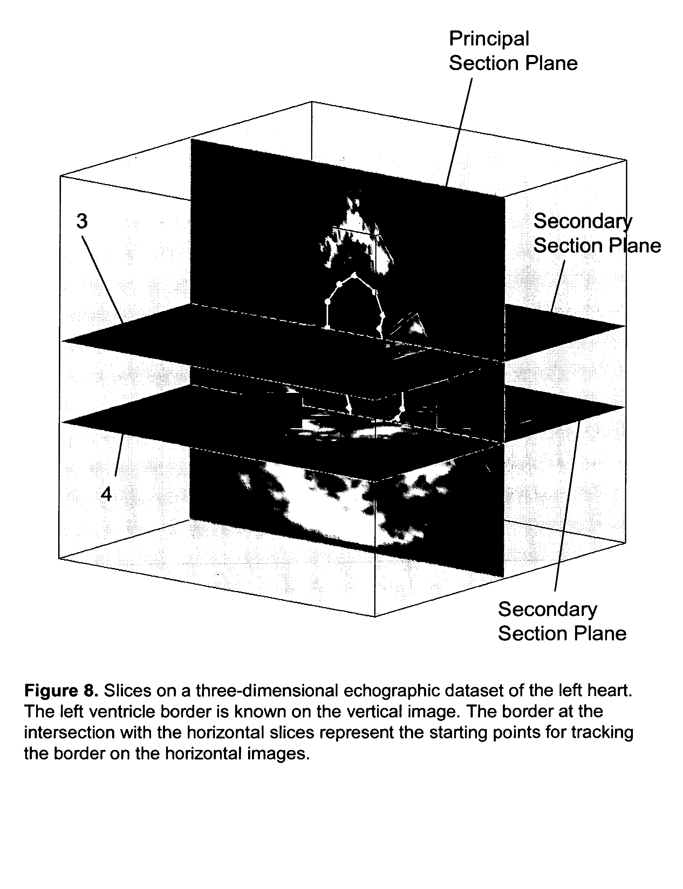

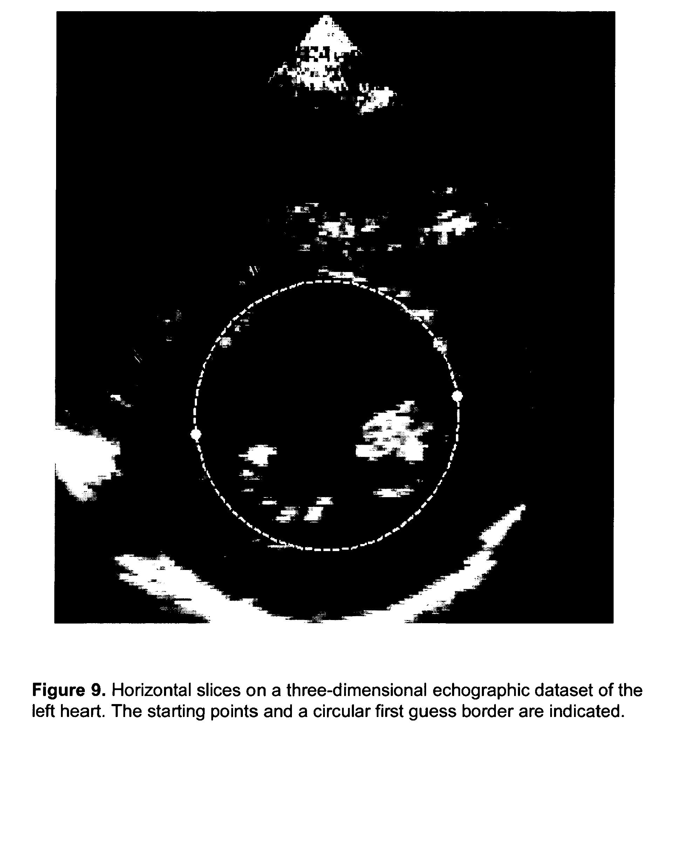

In each of such single M frames, the border now contains one or more reliable points, at the intersection with the principal section plane or planes 1, 2 and that come from the border(s) determined in the previous step relative to the principal section planes as illustrated in fig. 8 and indicated by R1, R2, R3, R4. A first guess border is constructed as a physiological relevant one passing through these reliable points R1, R2, R3, R4. An example of the said guess border on a secondary section plane is illustrated in the example of figure 9. Here the two dimensional image on a secondary section plane is illustrated together with the two reliable points R1 and R2. The guess border passing through the said two reliable points R1 and R2 is given by given by a circle in the transversal images of the left ventricle.

A new border is now detected by the same procedure used for a single transmural cut as disclosed in the previous chapter for the two dimensional case, this time however, substituting the time coordinate with the spatial coordinate along such first guess border as follows. Make a number of transmural cuts on the single image along the guess border, place the pixel found along each cut side by side in a new two-dimensional image and obtain a new image, like that in figure 4,
where the horizontal axis does not indicate the time coordinate but the spatial coordinate along the tentative border. As a result the correct border is tracked in one frame for each of the M sequences.

The above mentioned procedure is applied on all the sequences obtained from the appropriate cutting of the three-dimensional dataset according to each secondary section plane defined. The tracking technique disclosed above in the previous chapter of the two dimensional ultrasound imaging is applied to each of the M sequences taking as a starting, reliable, border that found on one frame in the step relating to the secondary section planes. The resulting M borders will define the complete surface border.

Similarly to the two dimensional case discussed above also in the three-dimensional imaging case the instantaneous velocity vector for a certain number of predefined points on the border surface can be calculated by using the same technique disclosed of the two dimensional case. The two dimensional technique disclosed above is used here by substituting the two dimensional estimate with a three-dimensional estimate of velocity.

When the tracking procedure is insufficient to define the entire velocity vector, this is done by selecting additional direction for the transmural cuts on the already displaced point and evaluating the velocity along the additional direction.

Alternatively, this is done by using a three-dimensional correlation or optical flow technique, in place of the two-dimensional one for evaluating the three-dimensional velocity vector.

Once the displacement vectors of the single landmarks or features represented by the reference points on the border line delimiting the region of interest are calculated, the inverse displacement can be determined and applied to the corresponding image of the sequence in order to displace the single reference point back in the position of the first image of the sequence of images. Further to this so called local registration from the vectors of displacements it is also possible to calculate a global evolution of the entire borderline and this relating either to the so called rigid displacement and to the deformations occurred to the borderline from the first to the following image for which the displacement of the reference points has been calculated.

Using for example one of the optical flow techniques it is possible to construct a transformation vector field for transforming back the region of interest and in this case the image of the ventricular wall from the condition of the following image in the condition of the first image and this relatively to the position and to the shape.

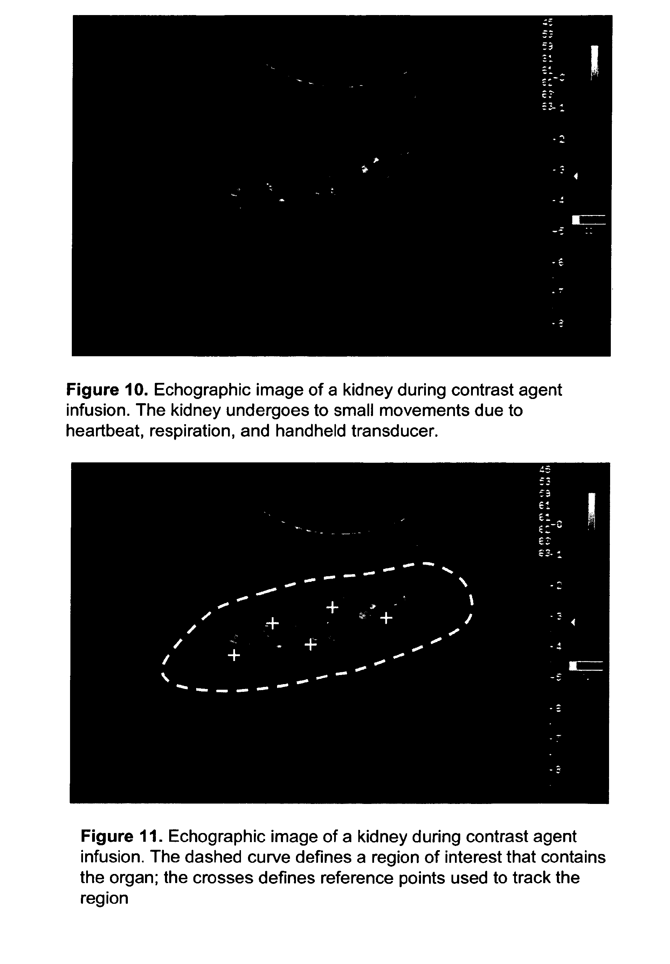

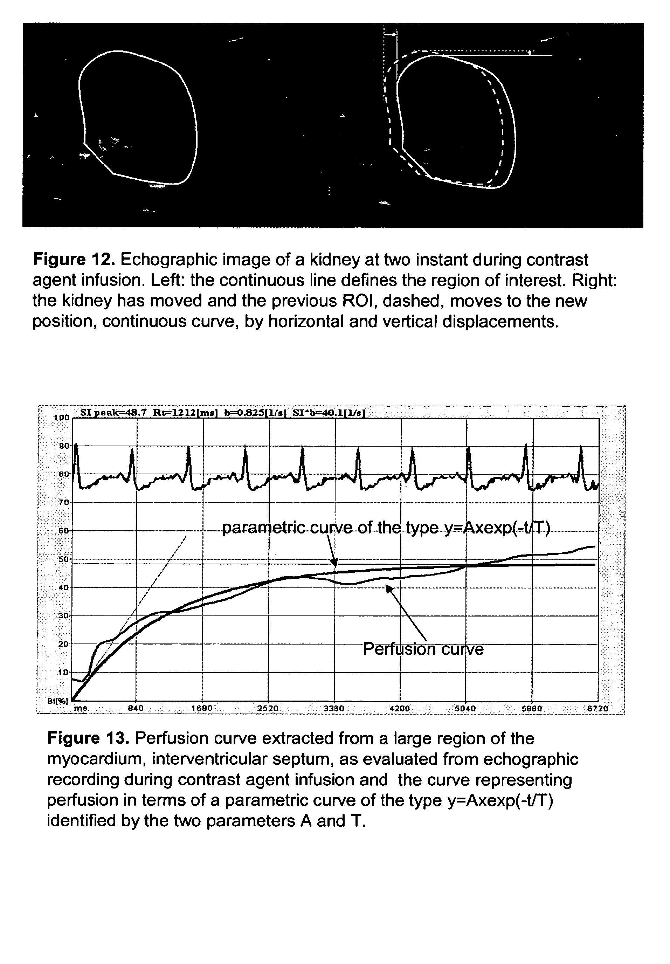

The examples of figures1 to 9 consider the situation in which the reference points being chosen as the landmarks to be tracked are selected on or near the borderline delimiting the region of interest. In the following example according to figures 10 to 12 a different organ is imaged. A sequence of images has been taken of the kidney. Image 10 is an echographic image of the kidney at a certain instant. Here the region of interest is represented by the image of the kidney. Thus as illustrated in figure 11 with a discontinuous line, a borderline is drawn either manually or automatically along the contours of the image of the kidney. In this example the reference points representing the landmarks or features to be tracked are chosen all inside the region of interest and not on the borderline delimiting this region. Nevertheless some of the reference points could have bean chosen also on the said borderline or outside the ROI. Tracking and determining the displacement vectors for each reference point can be performed by the PIV method or another optical flow technique. In the case of a three-dimensional organ, the tracking of reference points in three-dimensional space can be carried out in the same way by a three-dimensional PIV method or optical flow technique. Figure 12 illustrates the ultrasound images of the kidney at two different instants during contrast agent infusion. For sake of simplicity only a rigid displacement is illustrated as an example. In this figure the left hand image illustrates the borderline delimiting the image of the kidney as a continuous line. The right hand image has been acquired at a second following time instant. During the time interval occurred between the instant of acquisition of the first image and the instant of acquisition of the second image, the kidney has moved entirely from the position of the first left hand image to the position of the right hand image. This is represented in the right hand image by illustrating also the borderline in the original position of the left hand image and representing it with a discontinuous line. The displacement occurred along the horizontal and the vertical direction as it is illustrated by the displacement vectors oriented in the said two directions. Applying the inverse displacement vector the position of the kidney and thus of the chosen region of interest is restored as referred to the reference position considered as the one of the fist image of sequence of images. In other words displacement of the region of interest from the position in the first image to the position in the second, following image is compensated and the region of interest in the second following image is displaced back in a position identical to the one in the first image. This compensation can be applied not only to a linear rigid displacement of the region of interest, namely of the kidney, but the tracking information can be used also for determining an angular displacement and deformations of the region of interest, namely of the kidney which angular displacements and which deformations can also he compensated by applying the inverse angular displacement and deformation transform in the same way as disclosed for the rigid linear displacements.

From the above description it appears clearly the way in which tracking for registration purposes is carried out according to the registration method of the present invention. One of the most important effects is that the evolution of the entire ROI defining the organ/object under analysis, that is not a point-wise element but has an extension, is made of a combination of several dynamical movements like translation, rotation, deformations. These can be reconstructed on the basis of the computed displacements of the reference points in a conceptually same manner.

The rigid translation, the ROI displacement along the coordinates, the vertical and horizontal displacements in two-dimensional images (Figure 12), is evaluated by the displacements of the reference points by extracting their statistical most significant measure; for example the mean value or a weighted average, or the mean of the non extreme values, or the median, or another appropriate measure that may depend on the specific application.

The rotation about a given point can also be evaluated from the statistical significant measure of the rotation of each reference point. The deformation, as well as any other evolutionary characterization, can be equally extracted by evaluation of the corresponding relative displacements of the reference points.

In general the information about the reference points motion must be sufficient to evaluate the location of the ROI from one frame to any other in a way that the ROI, that moves, rotates, and deforms, can be identified on any frame of the sequence.

Tracking and registration have an important meaning for further image evaluation steps. These further evaluation steps are directed to extract information from the images of the sequence of images.

As a further step the present invention provides for an additional Step consisting in the extraction of the information relative to a moving region.

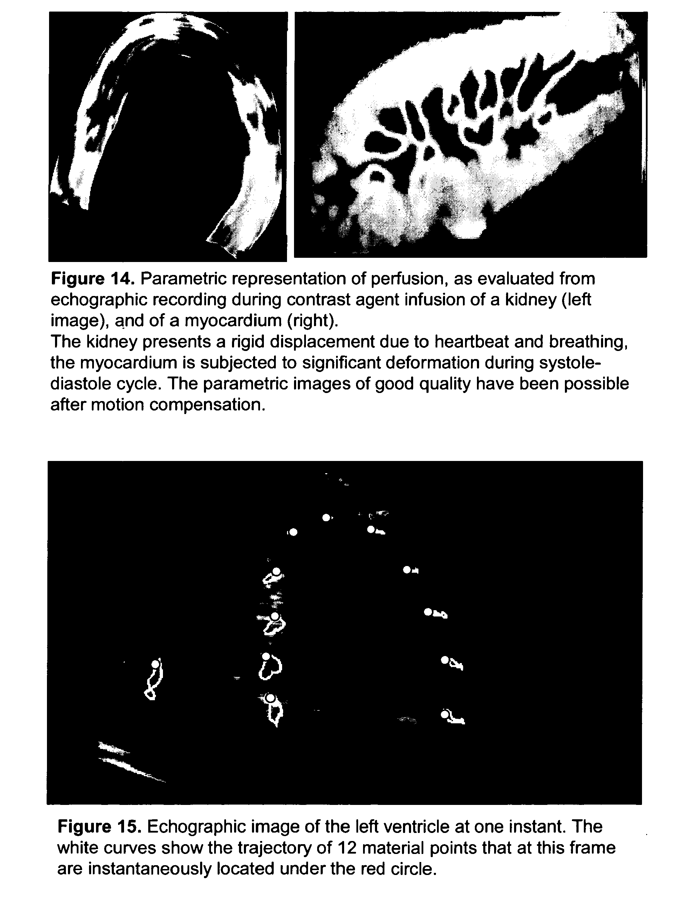

The information carried from the sequence of images is extracted in correspondence of the moving ROI. For example, in perfusion imaging, the change in brightness in any moving region inside the ROI gives a quantification of the microcirculation efficiency in such region. This method permits to compensate for the motion of the ROI. The information can be extracted as global measures for the entire ROI, like the average brightness or another datum, to give a time evolution of the information in the desired form (Figure 13). In other applications that require differentiation inside the ROI, the information can be extracted on each moving pixel to give rise to a parametric image of such quantity over the whole extension of the ROI (Figure 14). Parametric imaging methods are disclosed with greater details in document US 6,909,914 B2.

Perfusion measurements generally are such that reflect the contrast media time dependent behavior. Vascular and lymphatic flux provides for transporting contrast media in a certain anatomical district or organ and for transporting the contrast media away from a certain anatomical district. These physiological effects occurs within a certain time interval. Since the degree of vascularisation provides for a faster transport of the contrast media to or out of the anatomical district, the time dependent behavior of contrast media is an important way to measure the degree of vascularisation.

In perfusion measurements the ratio or the difference between the mean intensity or brightness of two images is calculated. Generally an image sequence of an organ or of an anatomical district is acquired. Each image of the sequence is acquired at a different time instant. Mean intensity of each image is calculated and can be presented in terms of a time dependent behavior or as the ratio of this mean intensity or brightness with the mean intensity or brightness of a reference (often a first) image of the sequence of images is calculated. The sequence of images are acquired at time instants during variation of the contrast media content in the anatomical district or in the organ he be imaged. Thus registration of the ROI for compensating motion and/or deformation of the ROI is important for optimizing perfusion results.

The present invention provides also further evaluation steps. In an embodiment where the dynamics of reference points is of interest the dynamic features and the trajectory of the reference points can be presented. This is useful for characterizing the kinematic behavior of the object or portion of tissue under analysis. Typically, trajectories of the myocardial tissue reflect the local contractility properties of the heart (Figure 15), in general a trajectory of material portion can be related with the function or dysfunction of that portion of tissue. Characteristics of such trajectories like orientation, length, area covered, etc. are a further method to synthetically describe them.

This quantification is another step that can be carried out independently from the presence or not of the other information extraction steps. In several situation it is useful -but not necessary- to perform this additional step with imaging of perfusion in order to have access to either the perfusion properties and the kinematic properties of a tissue. These together give a fairly complete description of the organ function.

A particularly suitable tracking method for extracting and visualizing the trajectories of the reference points, i.e. of the landmarks to be tracked is the so called PIV method which is described with greater detail in the already above cited published documents.

Alternatively also the Optical Flow tracking method can ensure optimum information for the trajectories of the reference points or landmarks to be tracked.

Although the invention is disclosed in combination of a special kind of tracking method using the above defined transmural cuts, other tracking methods may be used. As a first alternative tracking of the selected reference points or landmarks and extraction of the displacements relating to linear translations, rotations, deformations, and other kind of displacements may be carried out by using the known PIV method.

As a further alternative for tracking the reference points the so called Optical flow method can be used.

## Claims

1. Method for registering images of a sequence of images, particularly ultrasound diagnostic images and especially ultrasound diagnostic images of the heart. The said method comprising the steps of:
a) Providing at least a first and a second digital or digitalized image or set of cross-sectional images of the same object, the said images being formed by a two or three dimensional array of pixels or voxels;
b) Defining within one image of said set of images a certain number of landmarks, so called features, by selecting a certain number of pixels or voxels which are set as landmarks or features and generating a list of said features to be tracked;
c) Tracking the position of each pixel or voxel selected as a feature from one to another image of said set of images by determining the relative displacements for each pixel or voxel selected as a feature;
d) Registering the pair or set of images by applying the inverse displacement to the pixels or voxels between the images of said set of images.
**Characterized in that**
e) defining a region of interest, a so called ROI, by manually or automatically drawing a closed or open line on one image of the sequence of at least two images, which line corresponds to a trace of pixels or voxels of the said image and delimits the border of the said region of interest;
f) Defining one or more pixel or voxel which represents reference points within the region of interest defined by the said closed or open line and which reference points are the features or landmarks to be tracked;
g) for each one of the said features or landmarks estimating the displacement of the said feature or landmark from one image to a following or previous image starting form the first image by choosing a limited image region around the pixels or voxels defined as the landmarks or features and calculating the amount of local displacement of the said landmarks or features from a first to a second image of the sequence of images by determining the maximum likelihood between the said limited regions in the first and in the second image;
h) reconstructing the morphological and motion evolution of the entire region of interest by means of the local displacements calculated for each landmark or feature.
i) applying the inverse evolution function of the morphological features and of the motion of the entire region of interest to the second or to a following image of the sequence of images.

2. Method according to claim1, **characterized in that** the border line is drawn manually on the image.

3. Method according to claim1, **characterized in that** the border line is determined by means of an edge detection algorithm.

4. Method according to one or more of the preceding claims, **characterized in that** the pixels or voxels representing the reference points are chosen inside the region of interest, outside it, and/or on the borderline delimiting the said region of interest.

5. Method according to one or more of the preceding claims, **characterized in that** it provides for differentiated determination of the so called rigid translation and rotation of the entire region of interest and of the deformations of the entire region of interest.

6. Method according to one or more of the preceding claims, **characterized in that** the evolution in time of the entire region of interest is determined as a combination of several dynamical movements comprising translation, rotation, deformations, these movement being reconstructed on the basis of the computed displacements of the reference points.

7. Method according to claim 6, **characterized in that** the rigid translation, namely the displacement of the region of interest along the coordinates of a spatial reference system, i.e. the vertical and horizontal displacements in two-dimensional images, is evaluated by the displacements of the reference points by extracting their statistical most significant measure as the mean value or a weighted average, or the mean of the non extreme values, or the median, or another appropriate measure that may depend on the specific application.

8. Method according to claim 6, **characterized in that** the rotation about a given point is evaluated from the statistical significant measure of the rotation of each reference point i.e. of each landmark or feature.

9. Method according to claim 6, **characterized in that** the deformation is extracted from the statistical significant measure of deformation evaluated by the corresponding relative displacements of the reference points.

10. Method according to one or more of the preceding claims, **characterised in that** the line or the lines defining the border of the region of interest to be tracked is a line coinciding with an edge or a border of the image of an anatomical object or of an organ.

11. Method according to one or more of the preceding claims, **characterized in that** the pixels or voxels defined as reference points which has to be considered as landmarks or features to be tracked by a first reference image to a second or a following one of the sequence of images or frames are at least partly or entirely (all) chosen as lying on the said line defining the border of the image of the anatomical object or of an organ.

12. Method according to one or more of the preceding claims, **characterized in that** tracking of the reference points and of the border line is carried out by defining in each image frame of the sequence of images so called transmural cuts consisting in straight or curved lines, which are oriented transversally to the border line defining the region of interest, and passing through the pixel or reference points defined as a landmark or feature to be tracked and crossing the said border line.

13. Method according to claim 12, **characterised in that** the pixels taken along each transmural cut in each of the image frames of the sequence of image frames are placed in columns, each column corresponding to one image frame of the sequence of images the said columns representing the evolution along a transmural cut for all instants in a two-dimensional space time representation and the tracking of the border. i.e. of the trace of pixels is carried out along the space-time image using a cross-correlation procedure of the pixel column in the space-time image corresponding to a first image frame with the pixel column in the space-time image corresponding to a successive image frame of the sequence of image frames.

14. Method according to claims 12 or 13,
**characterised in that** the tracking of the border line is carried out by defining a certain number of reference points on the manually or automatically drawn border line on the first image frame and by defining for each reference point a line so called transmural cut which crosses the border line drawn on a first image frame and each of which lines passing through one reference point; each transmural cut line having a definite direction, which typically can be the orthogonal direction to the border line at the reference point;
carrying out the above mentioned step of defining transmural cuts for each image frame of the sequence of frames and for each reference point chosen;
the pixels taken along each transmural line in each of the image frames of the sequence of image frames being than placed in columns, each column corresponding to one frame of the sequence of images, forming a two-dimensional space-time representation of the evolution of the position of each reference point along the corresponding transmural cut;
the tracking of the border line i.e. of the trace of pixels, is carried out for each reference point defined on the said border line along the corresponding two-dimensional space-time image using a cross-correlation procedure of the pixel column in the space-time image corresponding to a first image frame with the pixel column in the space-time image corresponding to a successive image frame of the sequence of image frames.

15. Method according to claim 14, **characterised in that** The borderline is chosen as passing through some physiologically or anatomically relevant points of the imaged object and tracking is carried out by determining the motion of these few representative points prior to carry out the tracking of at least one or some of further reference points lying on the manually or automatically drawn border-line or inside the region of interest delimited by the said border line, in each frame of the sequence of image frames.

16. Method according to claim 15, **characterized in that** the following steps are provided for tracking:
a) Acquiring a sequence of at least two consecutive ultrasound image frames of a moving tissue or a moving object which ultrasound image frames are timely separated by a certain time interval;
b) Tracing a border line over one single first frame either manually or with the help of an automatic border drawing algorithm;
c) Tracking the position displacements of one or more eventually present representative reference points over the entire sequence of consecutive image frames;
d) Rescaling the border line drawn on the first image frame at least for some or for each of the following image frames of the sequence of image frames according to the corresponding position tracked of the representative reference points;
e) Defining a certain number of further reference points distributed along the border line on the first image frame and falling on the said border line;
f) Tracking the position of each point independently from the others along the sequence of image frames;
g) Tracking of the position of the representative reference points and of the other reference points being carried out by
h) for each point independently and in each of the image frames of the sequence of image frames defining a transmural cut line consisting in a line which crosses the border line drawn and passing through the said reference point;
i) the pixels taken along each transmural cut line in each of the image frames of the sequence of image frames are placed in columns, each column corresponding to one frame of the sequence of images for representing the evolution along a transmural cut line, for all instants at once in a two-dimensional space time representation;
j) the tracking of the border. i.e. of the trace of pixels along each transmural cut line is carried out along the space-time image using a cross-correlation procedure of the pixel column in the space-time image corresponding to a first image frame with the pixel column in the space-time image corresponding to a successive image frame of the sequence of image frames.

17. Method according to one or more of the preceding claims, **characterised in that** tracking and registration is applied to a sequence of three dimensional images according to the following steps:
l) Acquiring a sequence of three-dimensional ultrasound imaging data sets, each three-dimensional data set being acquired with a predetermined time interval from the previous one;
m) Defining at least a principal section plane of each three dimensional data set along one chosen direction for obtaining a sequence of two dimensional image frames along the said section plane;
n) Drawing a border line of the object imaged either manually or automatically on the first two dimensional image frame of the sequence of two dimensional image frames taken along the said section plane;
o) Carrying out the tracking steps c) to j) previously disclosed for the two dimensional sequence of image frames;
p) For each three dimensional data set of the sequence of three dimensional datasets defining a preestablished number of further secondary section planes crossing the at least one, preferably all the principal section planes, the said secondary section planes being spaced apart one from the other along a predetermined direction and dividing the object represented by each three dimensional data set of the sequence of three-dimensional data sets in slices.
q) For each secondary section plane in the sequence of three dimensional data sets constructing the corresponding sequence of image frames relative to the said secondary section plane;
r) For each sequence of two dimensional image frames determining a guess border line in one single frame, by letting the border line passing across the intersection points with the principal section planes;
s) Tracking the said guess border line by detecting a new border by applying the method steps according to c) to j) or e) to j) by substituting the time coordinate in the said disclosed steps with the spatial coordinate along the said guess border line by
t) defining a certain number of transmural cuts on the single image of the sequence of images along the guess border line;
u) identifying the pixels along the said transmural cuts and placing the pixels along each transmural cuts side by side for constructing a two dimensional image where the horizontal axis indicates the spatial coordinate along the guess border line;
v) carrying out the cross correlation between each of the consecutive pixel columns in the said two dimensional image and thus tracking the border line in one frame for each of the sequences of two dimensional image frames corresponding to each of the secondary section planes.

18. Method according to claim 17, **characterised in that** one or more further principal section planes can be defined along each of which further section planes the methods steps 1) to o) are carried out.

19. Method according to one or more of the preceding claims, **characterised in that** two orthogonal principal section planes are chosen for carrying out the above mentioned method steps, the crossing line of the two principal section planes defining a preferred direction of the said planes.

20. Method according to one or more of the preceding claims, **characterised in that** in order to better define the share of secondary section planes cutting the principal section planes, the steps are provided of defining bounds or limits for a distance range within which the share of the said secondary section planes is defined.

21. Method according to one or more of the preceding claims, **characterised in that** each reference point defined as a landmark or feature to be tracked and which displacements are tracked along the images of the sequence of images is visualized on the first image frame of the sequence of image frames together with a trace representing the path of displacement of the said reference points as determined by the tracking of its position in the images of the sequence of images.

22. Method according to one or more of the preceding claims, **characterised in that** the region of interest is the ventricular cavity, the region of interest to be tracked being defined by a border line which is manually drawn or automatically drawn as coinciding with the endoventricular wall and all or at least some of the reference points to be tracked being defined as pixels or voxels lying on the said border line.

23. Method according to one or more of the preceding claims, **characterized in that** the region of interest is the kidney and the border line coincides with the outer edge of the image of the kidney which is manually or automatically drawn, while all or at least some of the reference points are defined in the region delimited by the said border line and falling within the image area corresponding to the image of the kidney.

24. Method according to one or more of the preceding claims, **characterised in that** one reference image of the sequence of images acquired at different time intervals is acquired at a time when no or a certain amount of contrast agent is present in the imaged region or object while the other images of the sequence of images are acquired during variation of such amount of contrast agent present in the imaged region or object.

25. Method according to claim 24, **characterised in that** after tracking of the reference points in each of the images of the sequence the displacements of the said reference points at the position in the second or in one of the following images relatively to the position in the reference image are determined and the said displacement are compensated by registering the said images of said sequence, and the intensity of the obtained registered images is calculated being associated univoquely to the time instant o acquisition of the corresponding image and a graphic representation of the behaviour in time of the intensity of the image is represented as a curve.

26. Method according to one or more of the preceding claims, modified in that the tracking steps are carried out by applying the PIV method.

27. Method according to one or more of the preceding claims 1 to 25, modified in that the tracking steps are carried out by applying the Optical Flow method.
